Europäisches Patentamt

(19) European Patent Office

Office européen des brevets

(11) Publication number: **0 014 866**
**B1**

(12) **EUROPEAN PATENT SPECIFICATION**

(45) Date of publication of patent specification: **26.01.83**

(51) Int. Cl.³: **C 12 N 11/08, C 12 P 19/24**

(21) Application number: **80100449.0**

(22) Date of filing: **29.01.80**

(54) **An immobilized glucose isomerase system and a process for the isomerization of glucose using said system.**

(30) Priority: **30.01.79 US 7550**

(43) Date of publication of application:
**03.09.80 Bulletin 80/18**

(45) Publication of the grant of the patent:
**26.01.83 Bulletin 83/4**

(84) Designated Contracting States:
**DE IT**

(56) References cited:
**DE - A - 2 918 929**
**GB - A - 1 557 944**
**US - A - 3 960 663**

**DIE STÄRKE, vol. 27, no. 9, 1975, pages 302—306 Y. YOKOTE et al.: "Production of high fructose syrup by glucose isomerase immobilized on phenolformaldehyde resin"**
**PATENTS ABSTRACTS OF JAPAN, VOL. 2, NO. 122, 13th OCTOBER 1978, PAGE 2403C78**

(73) Proprietor: **CPC INTERNATIONAL INC.**
**International Plaza P.O. Box 8000**
**Englewood Cliffs New Jersey 07632 (US)**

(72) Inventor: **Walon, Raoul G.P.**
**25 Avenue d'Hulderghem rez-de-chaussée**
**B-1020 Brussels (BE)**
Inventor: **Stouffs, Robert H. M.**
**336 Boulevard De Souverain**
**B-1160 Brussels (BE)**

(74) Representative: **Lederer, Franz, Dr. et al,**
**Patentanwälte Dr. Franz Lederer Dipl.-Ing. Reiner**
**F. Meyer-Roxlau Lucile-Grahn-Strasse 22**
**D-8000 München 80 (DE)**

Courier Press, Leamington Spa, England.

# 0 014 866

An immobilized glucose isomerase system and a process for the isomerization of glucose using said system

This invention relates to an immobilized glucose isomerase system and to a method of isomerizing glucose.

Glucose isomerase is the general name for the enzyme class which converts glucose (dextrose) into levulose (fructose). Since glucose isomerase was discovered as a product of microorganisms, the industrial production of levulose-bearing syrup from glucose on an industrial scale has become a major commercial process.

While levulose-bearing syrup can be prepared in a batch system using cells containing glucose isomerase, immobilized enzymes are preferred because they facilitate continuous as opposed to batch processing. Thus, methods of immobilizing glucose isomerase on various supports have been proposed and described in the prior art.

For example, immobilization of glucose isomerase on certain inorganic carriers is described in U.S. Patent Nos. 3,556,945 and 3,992,329.

The use of cellulose derivatives and synthetic resins as supports for glucose isomerase has also been described in the literature. Thus, U.S. Patent No. 3,909,354 describes the conversion of glucose to levulose using glucose isomerase bound to either an anion exchange cellulose or a synthetic anion exchange resin. U.S. Patent No. 4,078,970 discloses glucose isomerase adsorbed and bound on an anion exchange resin having a porosity of more than 4.5% measured according to an aqueous dextrose solution method and an ion exchange capacity of more than 0.035 meq/ml resin measured according to a polyanion salt decomposition method. The resins are prepared by suspension polymerization of styrene, divinyl benzene, polystyrene and toluene in water using benzoyl peroxide as a catalyst, followed by chloromethylation and introduction of anion exchange groups. Japanese Specification No. NS80160/74 describes the immobilization of glucose isomerase on specific polyphenol resins, while Japanese Patent Specification No. S48—56770 describes the use of triazine derivatives to bind enzymes to polystyrene, polyphenol, alkylene, polyamine and aliphatic amine ion exchange resins. In Belgian Patent No. 862,765, a macroporous enzyme support having no ionic characteristics is described.

While many of the previously described supports for glucose isomerase do function to produce levulose-bearing syrups, they possess certain disadvantages. For example, some carriers result in poor enzyme activity, while other carriers require low throughput. Furthermore, many of these carriers are expensive and some must be regenerated and reused several times, adding to manufacturing costs.

Notwithstanding the plethora of enzyme supports known in the prior art, it has been discovered that a phenolic resin having a specific porosity profile and water swelling capacity has surprising and unexpected properties when used as a support for a specific glucose isomerase.

More in detail, this invention is directed to an immobilized glucose isomerase system that includes a glucose isomerase derived from *Streptomyces olivochromogenes* ATCC Nos. 21,114; 21,713; 21,714 or 21,715. Deposited strains ATCC Nos. 21,713; 21,714 and 21,715 are mutant strains of *Streptomyces olivochromogenes* ATCC No. 21,114. The glucose isomerase can also be derived from mutant strains of the ATCC No. 21,715 microorganism. A commercially available glucose isomerase that is derived from the ATCC No. 21,715 microorganism or mutant strains thereof is sold under the name of G-993 by CPC International Inc., International Plaza, Englewood Cliffs, New Jersey, U.S.A. The glucose isomerase is immobilized on a porous phenolic resin wherein said resin has a total surface area of 40 to 200 square meters per gram in the range of pores above 30Å of which at least 30% of the pores have a radius of 30 to 250Å and a water swelling capacity of at least 10% by volume and wherein said system has a binding efficiency of at least 90% when an enzyme load of 500 U/ml carrier is offered or at least 80% when an enzyme load of 1000 U/ml carrier is offered, and an enzyme half-life during isomerization of at least 10 days.

Preferably the porous phenolic resin is an anion exchange type resin. Preferred resins are those, in which of the pores having a radius of 30 to 250Å at least 50% have a radius of 50 to 150Å, and/or at least 20% have a radius of 60 to 90Å, and/or at least 20% have a radius of 70 to 80Å.

This invention is directed to a single use system which provides high binding capacity for glucose isomerase, high flow rates, long column lives and low enzyme consumption, thus making it extremely attractive for use in an economical commercial process.

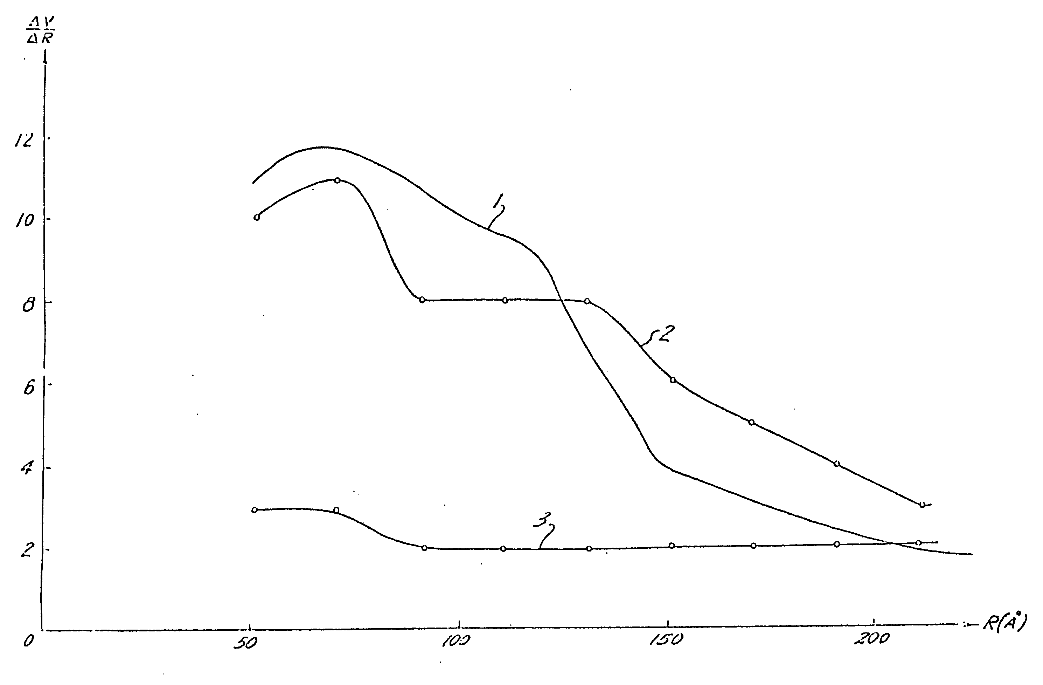

The Figure is a differential pore radius distribution curve of several carriers, including the carrier of this invention.

More in detail, the porous phenolic resin carriers defined in the claims have a porosity profile similar to curve 1 in the Figure. This curve was obtained by measuring pore size and volume by mercury porosimetry, plotting an integral curve expressing the relation between the volume condensed in the pores and the radii, and differentiating the integral curve to obtain the more illustrative differential distribution curve which expresses the increment of pore volume corresponding to a differential increase in their radius. A detailed description of this measurement together with a description of the measurement of surface area, is set forth in "Adsorption On Solids", V. Ponec, et al., Butterworths, 1974, pages 38—39, 540—545 and 552—555.

**0 014 866**

While any phenolic resin having the surface area and porosity defined herein can be used in the practice of this invention, it is preferred to employ a resin having an anion exchange capacity. Specific resins found to be useful are Duolite ES-562 and Duolite ES-568. These are phenol-formaldehyde resins available from Diamond Shamrock.

The specific glucose isomerase enzyme employed in the practice of the invention is derived from *Streptomyces olivochomogenes* ATCC Nos. 21,114; 21,713; 21,714 or 21,715. Deposited strains ATCC Nos. 21,713; 21,714 and 21,715 are mutant strains of *Streptomyces olivochromogenes* ATCC No. 21,114. The glucose isomerase can also be derived from mutant strains of the ATCC No. 21,715 microorganism. As pointed out earlier in this specification, a commercially available glucose isomerase that is derived from the ATCC No. 21,715 microorganism or mutant strains thereof is sold under the name G-993 by CPC International Inc., International Plaza, Englewood Cliffs, New Jersey, U.S.A. The liquid enzyme concentrate is made in accordance with the procedure described in U.S. Patent No. 4,077,842. Regarding the activity of the enzyme, one unit (referred to as U) is defined as the amount of enzyme which produces one micromole of levulose per minute, in a 0.1 molar solution of glucose at pH 7.5 and 60°C in the presence of 0.01 molar $MgCl_2$ and 0.001 molar $CoCl_2$, using the following assay procedure, involving making a spectrophotometric determination of the ketose produced from a glucose solution under a standardized set of conditions.

A stock solution is made up in the following manner:

| | Stock solution for assay | |
| Component | | Amount |
|---|---|---|
| 0.01 M $MgCl_2$ | | 1 ml |
| 0.001 M $CoCl_2$ | | 1 ml |
| 1.0 M Phosphate buffer, pH 7.5 | | 0.5 ml |
| Anhydrous D-glucose | | 1.44 g. |
| Distilled water | | To make up a total volume of 7.5 ml. |

The enzyme preparation to be assayed is first diluted to contain from 1 to 6 U/ml.

An enzymatic isomerization is conducted by adding 1 ml of the enzyme preparation to 3 ml of the stock solution, and incubating for 30 minutes at 60°C. At the end of the incubation period, a 1 ml aliquot is taken and quenched in a 9 ml volume of 0.5 N perchloric acid. The quenched aliquot is then diluted to a total volume of 250 ml. As a control, for comparative purposes, a glucose blank is also run by substituting 1 ml of water for 1 ml of the enzyme preparation in solution form, at the beginning of the incubation period. The ketose is then determined by a cysteinesulfuric acid method. For the purposes of this assay, one U is defined as the amount of enzyme activity that is required to produce one micromole of levulose per minute under the isomerization conditions described above.

In order to prepare the immobilized system of this invention, the porous phenolic resin is mixed with glucose isomerase solution consisting of between about 100 and 1000 units of enzyme solution per ml of dry resin carrier. Preferably the enzyme preparation is very concentrated and contains from about 1000 to about 2500 Units per ml of enzyme solution. The porous resin is first washed, preferably with demineralized water, until it has swollen at least about 10% by weight. Then, the resin is treated to adjust the pH to between 7.0 and 9.0, preferably between 7.5 and 8.5. It is preferred to employ a caustic wash step prior to the final pH adjustment in order to obtain a product pH of close to 8.2, thus optimizing the conversion to levulose. After the desired pH has been obtained, the enzyme adsorption step is carried out for at least 20 hours, depending on such factors as enzyme solution concentration and the like. A final wash may be carried out at this point to insure that the pH is still within the desired range. Once the enzyme is immobilized, the resulting system can be used immediately, or stored either in water or as a moist system until used.

In use, the system is reacted with a starch hydrolysate solution. The term "starch hydrolysate" is used to refer to a syrup or dry product that is made by acid and/or enzymatic hydrolysis of starch. A preferred type cf starch hydrolysate for use for isomerization in accordance with the present invention is produced by acid or enzyme thinning to a D. E. of 20 or less followed by enzymatic saccharification to a D. E. above 90, preferably about 95. The term "D. E." is an abbreviation of "dextrose equivalent" and refers to the reducing sugar content of the material, calculated as dextrose and expressed as percent of total solids. During the saccharification process, the pH should be in the range between 4.0 and 5.0. Various buffers known to those skilled in the art may be used to achieve the desired pH.

In the preferred continuous process, the porous phenolic resin particles are placed in a plugged flow-through column and the enzyme immobilization procedure carried out prior to passing the starch hydrolysate through the column. Preferably the concentration of the starch hydrolysate solution is at least about 30% by weight. Flow rate, which is referred to as BVH, is adjusted during the process to assure maximum isomerization, e.g., about 45% conversion of the glucose to fructose. BVH is the column flow rate in bed volumes per hour.

3

In an alternative embodiment of the process of this invention, between 50 to 600 ppm of $SO_2$ is added to the starch hydrolysate supply. Preferably at least 300 ppm $SO_2$ is added in order to enhance the stability of the glucose isomerase.

The following examples will serve to illustrate the practice of this invention.

Example 1

*Streptomyces olivochromogenes* (ATCC No. 21,715 was shake-cultured in liquid culture medium at 30°C for about 50 hours. The cultured material was then centrifuged for 20 minutes at 10,000 rpm to collect the cells. The wet cells were weighed and then suspended in ion exchanged water (3 volumes) containing 10 mM $MgCl_2$. Next 0.02% of lysozyme (a product of Boehringer-Mannheim Co.) and 1% toluene by weight of wet cells were added and lysis was carried out for 24 hours at 30°C under gentle stirring. The resulting lysate was then centrifuged for 20 minutes at 10,000 rpm and the cellular debris eliminated.

An equal volume of isopropyl alcohol was next added to the supernatant while it was being gently stirred. This mixed solution was further centrifuged and the precipitate collected. The precipitate was then dissolved in a small amount of deionized water containing 10 mM $MgCl_2$. The performance of the above series of operations resulted in a recovery of over 70% of the initial, intracellular glucose isomerase in a soluble form.

Pore size and volume of Duolite ES-562, a phenol formaldehyde resin available from Diamond Shamrock Corp., Painesville, Ohio, was measured using a Mercury Pressure Porosimeter Model 200 available from Carlo Erba Strumentazione, Milan, Italy. The results are represented by curve 1 of the Figure. The specific surface area as determined by the BET method was 100 $m^2$/gm. Then 10 ml of the resin was washed in a beaker with 100 ml of 0.1 M citric acid (pH 1.5) for one hour under intermittent agitation. The citric acid was removed by decantation. The resin was then washed twice with hot demineralized water to remove air bubbles. The same procedure was repeated four times to provide four batches of resin.

Four 10 ml columns were filled with 10 ml of treated resin and again the resin was washed downflow with demineralized water at 4 BVH for 2 hours. The four columns were connected to the same waterbath and to one single multi-channel peristaltic pump. Four columns were used to avoid any discrepancies due to variations in temperature and/or flow rate.

The resin was then washed with 0.1 M magnesium acetate (pH 7.4) for one hour at 4 BVH.

The previously mentioned glucose isomerase solution was offered at an enzyme load of 480 U/ml carrier to each column and recirculated for 16 hours at a flow rate of 4 BVH in order to immobilize on the resin. Then, the resin was washed with 0.1 M magnesium acetate (pH 7.4) at 4 BVH till a total volume of 40 ml effluent was available for bound enzyme measurement.

Then, the columns were fed with a pure dextrose solution at 50% d.s. containing 200 ppm Mg++ as magnesium sulfate and adjusted with sodium hydroxide to a pH of 8.4. A constant flow rate of 4 BVH was applied. After one hour, the column temperatures were raised to 60°C. A first sample of isomerized liquor was taken from each column after one hour elution at 60°C and the levulose content was measured by polarimetry. A second sample was taken after 20 hours of continuous isomerization.

The equations given below were then used to calculate the amount of bound enzyme, that is, the percent of glucose, isomerase bound to the resin and the bound enzyme activity. The bound enzyme activity was measured as BVH 45% after one hour and 20 hours.

$$\text{Bound enzyme, } \% = 100 - \frac{(\text{U/ml measured in effluent} \times 40 \times 100)}{(\text{enzyme offered, U.})}$$

$$\text{BVH } 45\% = \frac{\text{BVH}_x \ \% \ \log \dfrac{51.2}{51.2-x}}{.916}$$

where x=actual conversion.

For the four columns, a binding of 96% was determined, with an activity of 3.7 BV after one hour and 7.0 BV after 20 hours, representing outstanding binding capacity and activity.

Examples 2—18

In order to confirm the percent binding and the activity of a resin carrier in accordance with this invention, several experiments were carried out in accordance with the following general procedure.

In Examples 2—9, columns having dimensions of 2.5×30 cm were packed with 100 mls of Duolite ES-562 resin. Then, the resins were washed with demineralized water at 60°C for one hour to remove any entrapped air bubbles. Next, the packed resins were washed with 0.1 M magnesium acetate (pH 7.5) at 25°C for 1.5 hours at a rate of 4 BVH. Then, the glucose isomerase solution described in Example 1, with an enzyme load as indicated, was added to each column at a rate of 4 BVH and recirculated for 24 hours. After that, the resins were washed with 0.1 M magnesium acetate (pH 7.4) at a rate of 4 BVH for 3/4 hour and the effluent collected for bound enzyme determination in accordance with the procedure described in Example 1.

Then, the columns were fed with a solution containing 97 DE hydrolysate at 50% d.s. and magnesium sulfate at 200 ppm Mg++ buffered with sodium hydroxide to the indicated pH. Some of the feeds contained $SO_2$ as a stabilizer for the enzyme. An initial flow rate of 6 BVH was applied. After one hour, the column temperatures were raised to 60°C and the flow rates adjusted to achieve a continuous isomerization rate (isomerization of the dextrose into 42% levulose). TABLE 1 shows the percent binding, the initial BVH rate for 45% conversion, the first and second half-lives, the total bed volumes used and the enzyme consumption. Enzyme consumption is reported in units per gram of dry product (u/g d.s.).

In Example 13, the enzyme was added to the pre-treated resin in amounts as described above but in a batch system and the resulting carrier-enzyme was completely dried at room temperature prior to introduction into the column. It appeared that the drying resulted in partial denaturation of the enzyme.

TABLE I

| Examples | 2 | 3 | 4[1] | 5[1] | 6[2] | 7 | 8 | 10 | 11 | 12 | 13[3] |
|---|---|---|---|---|---|---|---|---|---|---|---|
| Enzyme offered (U/ml carrier) | 480 | 240 | 480 | 480 | 240 | 240 | 480 | 480 | 480 | 480 | 480 |
| pH | 8.4 | 7.6 | 8.4 | 8.4 | 7.6 | 7.6 | 8.4 | 8.4 | 8.4 | 8.4 | 8.4 |
| $SO_2$ ppm | — | 400 | — | — | 400 | 400 | — | 50 | 100 | 200 | — |
| % Binding | 98.3 | 99.6 | 26.9 | 27.8 | 99 | 99 | 94.3 | 95.2 | 95.7 | 95.8 | 91.8 |
| Initial BVH 45% conversion | 6.8 | 3.7 | — | — | 2.8[6] | 2.7[6] | 4.2 | 6.0 | 6.0 | 6.0 | 1.8 |
| 1st Half life (days) | 34.6 | 35.4 | — | — | — | — | — | 35.5 | 35.5 | 35.5 | — |
| 2nd Half life (days) | 17.1 | 116.7 | — | — | — | — | — | 18.75 | 18.75 | 18.75 | — |
| Total life (days) (sum of 1st and 2nd half lives) | 51.7 | 152.1 | — | — | — | 62.5 | 30.2 | 54.25 | 54.25 | 54.25 | — |
| Total BV | 5532 | 6300 | — | — | — | 4050 | 3045 | 4612 | 4612 | 4612 | — |
| Enzyme consumption (u/g d.s.) | 0.15 | 0.06 | — | — | — | 0.097 | 0.26 | 0.17 | 0.17 | 0.17 | — |

TABLE 1 (Continued)

| Examples | 14 | 15 | 16[4] | 17[5] | 18[8] |
|---|---|---|---|---|---|
| Enzyme offered (U/ml carrier) | 240 | 240 | 240 | 240 | 480 |
| pH | 8.4 | 8.4 | 8.4 | 8.4 | 7.6 |
| $SO_2$ ppm | — | 400 | — | — | — |
| % Binding | 100 | 100 | 100 | 100 | 92.4 |
| Initial BVH 45% conversion | 4.5 | 4.0 | 4.5 | 5.4 | 5.0 |
| 1st Half life (days) | 33.3 | 35.8 | 35.0 | 29.2 | 38.5 |
| 2nd Half life (days) | 18.8 | 18.3 | 18.3 | — | 26.1 |
| Total life (days) ($\rightarrow$ 0.5 BVH)[7] | 60.4 | 62.5 | 62.5 | — | 77.0 |
| Total BV | 3625 | 3450 | 3750 | — | 5088 |
| Enzyme consumption (u/g d.s.) | 0.11 | 0.11 | 0.11 | — | 0.15 |

[1] Runs with reused carrier; because sufficient enzyme did not bind, the experiment was discontinued.

[2] Because the hydrolysate was of poor quality, i.e., high color and low dextrose content, the experiment was discontinued.

[3] Because the bed volume was too low, the experiment was discontinued.

[4] Magnesium acetate was added in an amount representing 100 ppm Mg++.

[5] The feed was 97 D.E. hydrolysate at 45% solids.

[6] Content flow-rate, variable temperature (55—65°C) applied for 45% conversion; therefore, half-lives cannot be given.

[7] Total life in these Examples represents the total operation time until 0.5 BVH.

[8] Feed was not demineralized but decalcified.

Examples 19—22

The general procedure of Examples 2—18 was followed using G-993 glucose isomerase with the exception that the columns were half-filled with demineralized water at 60°C prior to packing, and that after the water washing the resin was washed with 2% NaOH at 25°C for 2 hours at a rate of 2 BVH followed by water washing at 25°C for 15 hours at 2 BVH in order to adjust the pH to about 8. Then, the resin was washed with 0.1 M magnesium chloride (pH 7.5) at 25°C for 1-1/2 hours at a rate of 4 BVH. Enzyme loading was carried out as described in Examples 2—18 except that G-993 glucose isomerase was used. This was followed by washing with magnesium chloride at 25°C for 3/4 hour at 4 BVH. The effluent was collected for bound enzyme determination. Then, the feed solution was added as described in Examples 2—18; the results are set forth in TABLE 2. (In Example 20, the feed solution was decalcified but not demineralized. In Example 22, a saturated solution of sodium bisulfite was added to the feed to provide approximatey 200 ppm $SO_2$).

TABLE 2

| Example | 19 | 20 | 21 | 22 |
|---|---|---|---|---|
| Enzyme offered (U/ml carrier) | 480 | 480 | 480 | 480 |
| pH | 8.4 | 8.4 | 7.6 | 7.6 |
| % Binding | 96.5 | 90.6 | 74.7 | 91.0 |
| Initial BVH 45% conversion | 5.7 | 4.9 | 5.0 | 6.2 |
| 1st Half life (days) | 33.3 | 41.2 | 37.1 | 52.1 |
| 2nd Half life (days) | 20.9 | 19.2 | 19.2 | 60.4 |
| Total life (days) (→0.5 BVH) | 66.7 | 75.0 | 66.7 | 146 |
| Total BV | 4800 | 4680 | 4400 | 9975 |
| Enzyme consumption (u/g d.s.) | 0.16 | 0.17 | 1.18 | 0.078 |

Example 23

In order to demonstrate the superior properties of the resin carrier of this invention, experiments were carried out using the Duolite ES-562 and a porous $Al_2O_3$—MgO support material having an average pore diameter ranging from 100Å to 1000Å and made in accordance with the process described in U.S. Patent No. 3,992,329. The equipment and procedure of Example 1 were followed; the results are set forth in TABLE 3 below. These results demonstrate that the carrier of this invention has a higher binding capacity, a higher initial BV rate and a longer column life than the $Al_2O_3$—MgO support. Furthermore, these properties result in lower enzyme consumption for the carrier of this invention, the Duolite ES-562 required only 64% of the enzyme consumed by the $Al_2O_3$—MgO support.

TABLE 3

| Carrier | Duolite ES-562 | $Al_2O_3$—MgO |
|---|---|---|
| | | Average* |
| Bound enzyme, % | 98.7 | 94.8 |
| Initial BVH 45% | 7.0 | 5.5 |
| 1st Half life (days) | 29.1 | 29 |
| 2nd Half life (days) | 32.3 | 15 |
| Total life (days) (sum of 1st and 2nd half lives) | 61.4 | 44 |
| Total BV | 5729 | 3500 |
| Enzyme consumption on offered, u/g d.s. | .136 | .22 |

*Average of several columns; deviation among columns varied from .21 to .23 u/g d.s.

Example 24

In order to ascertain the influence of the amount of offered enzyme, various amounts of enzyme were offered to the Duolite ES-562 carrier. The equipment and procedures of Examples 19—22 and the enzyme of Example 1 were employed, varying only the amount of enzyme. The results, as set forth in TABLE 4A, show the high binding capacity of this resin.

## 0014866

### TABLE 4A

| Enzyme offered (U/ml carrier) | 240 | 480 | 960 |
|---|---|---|---|
| Bound enzyme, % | 99.5 | 98.7 | 88.9 |
| Initial BVH 45% | 4.2 | 7.0 | 12 |
| 1st Half life (days) | 31.3 | 29.1 | 20.8 |
| 2nd Half life (days) | 31.2 | 32.3 | 29.2 |
| Total life (days) (sum of 1st and 2nd half lives) | 62.5 | 61.4 | 50.0 |
| Total BV | 3525 | 5729 | 7650 |
| Enzyme consumption on offered, u/g d.s. | .111 | .136 | .204 |

The same procedure was followed using the $Al_2O_3$—MgO carrier; the results are set forth below in TABLE 4B. As this comparative data clearly shows, the highest initial BV with this carrier was 7.3, and the enzyme consumption decreased with enzyme load. Also, at a loading of 480 U/ml carrier, the Duolite ES-562 delivers the same initial BV rate as the $Al_2O_3$—MgO loaded with 624 U/ml carrier.

### TABLE 4B

| Enzyme offered (U/ml carrier) | 750 | 624 | 480 | 336 | 240 |
|---|---|---|---|---|---|
| Bound enzyme, % | 70.4 | 75.7 | 80 | 84.4 | 89.4 |
| Initial BVH 45% | 7.3 | 7.0 | 5.35 | 3.9 | 3.2 |
| 1st Half life (days) | 23.8 | 25 | 27.1 | 25 | 28.1 |
| 2nd Half life (days) | 12.4 | 11.5 | 13.5 | 12.7 | 11.9 |
| Total life (days) (sum of 1st and 2nd half lives) | 36.2 | 36.5 | 40.6 | 37.7 | 40.0 |
| Total BV | 3915 | 3850 | 3270 | 2330 | 2020 |
| Enzyme consumption on offered, u/g d.s. | .300 | .264 | .239 | .235 | .194 |

Example 25

In this example, runs were made under conditions which included adding $SO_2$ to the liquor supply in order to increase the stability of the glucose isomerase enzyme. The equipment and procedure of Examples 19—22 and the enzyme of Example 1 were employed with the following changes:

(a) 400 ppm $SO_2$ were added to the feed of 97 DE hydrolysate;

(b) 240 U/ml carrier of enzyme were offered to the carrier; and

(c) the liquor supply was regulated at pH 7.6.

TABLE 5 shows that although the addition of $SO_2$ extends column life for both Duolite ES-562 and the $Al_2O_3$—MgO support described in Example 24, the former results in approximately a 29% reduction of enzyme consumption when compared to the latter.

### TABLE 5

| Carrier | Duolite ES-562 | $Al_2O_3$—MgO |
|---|---|---|
| Enzyme offered, U/ml carrier | 240 | 240 |
| Bound enzyme, % | 99.6 | 98.8 |
| Initial BVH 45% | 3.7 | 3.2 |
| 1st Half life (days) | 35.4 | 20.8 |
| 2nd Half life (days) | 116.7* | 114.5* |
| Total life (days) | 152.1* | 135.3* |
| Total BV | 6300* | 4500* |
| Enzyme consumption on offered, u/g d.s. | .062* | .087* |

*Results were extrapolated after 79 days of continuous operation; at that time, the enzyme consumption of $Al_2O_3$—MgO was .12 u/g d.s.; for Duolite ES-562, the enzyme consumption was .098 u/g d.s.

Examples 26—32

In order to demonstrate that the system and process of this invention can be used on a plant scale, runs were made in columns 3 meters high and having a diameter of 50 cm. The previously mentioned glucose isomerase solution based on G-993 glucose isomerase was added to Duolite ES-562, which had been pre-treated with sodium hydroxide in a batch system.

Each of the columns was then packed with 600 liters of the resulting carrier-enzyme system. Then, the columns were fed with a 97 DE hydrolysate solution at 50% d.s. containing 200 ppm Mg++ as magnesium sulfate and adjusted with sodium hydroxide to a pH of 8.4. The initial column temperature was 53°C. Since a relatively constant throughput was desired, the columns were operated by means of a compromise between a constant temperature and a variable temperature process. Thus, a comparison between lab and plant scale runs are difficult and meaningful half-lives could not be determined. The following results, which are illustrative of the plant scale runs, are given merely to show the feasibility of scaling-up this invention.

TABLE 6

| Examples | 26 | 27 | 28 | 29 | 30 | 31 | 32 |
|---|---|---|---|---|---|---|---|
| Enzyme offered (U/ml carrier) | 348 | 373 | 281 | 377 | 383 | 382 | 383 |
| % Binding | 73 | 82 | 32 | 81 | 72 | 81 | 88 |
| Initial BVH 45% | 3.95 | 3.33 | — | 3.13 | 2.70 | 2.80 | 2.83 |
| Days of operation | 92 | 50 | 40 | 54 | 70 | 57 | 47 |
| Enzyme consumption (u/g d.s.) | 0.124 | 0.22 | 0.36 | 0.25 | 0.23 | 0.28 | 0.29 |

Comparative Examples

In order to demonstrate the efficacy of the resin carrier of this invention, experiments were carried out using resins having a porosity profile different from that of the carriers of this invention. These resins were Duolite ES-762, a phenolic resin having the porosity profile represented by 2 in the Figure, and Duolite A-7 Fines, a resin having the porosity profile represented by 3 in the Figure. Both resins are available from Diamond Shamrock Corp. The results are set forth in TABLE 6 below, with the Duolite A-7 Fines resulting in such a low initial BVH 45% that the run was discontinued. The Duolite ES-762 had a poor life and high enzyme consumption. G-993 glucose isomerase was used as the enzyme.

TABLE 7

| Carrier | Duolite ES-762 | Duolite A-7 Fines |
|---|---|---|
| Enzyme offered, U/ml carrier | 480 | 480 |
| Bound enzyme, % | 92.3 | 80.8 |
| Initial BVH 45% | 3.5 | 1.2 |
| 1st Half life (days) | 9.2 | — |
| 2nd Half life (days) | 22.1 | — |
| Total life (days) (sum of 1st and 2nd half lives) | 31.3 | — |
| Total BV | 1155 | — |
| Enzyme consumption on offered, u/g/d.s. | 0.68 | — |

A run was also attempted with Duolite A-368/S, but with the BVH so low that the experiment wa discontinued.

**Claims**

1. An immobilized glucose isomerase system comprising glucose isomerase derived from *Streptomyces olivochromogenes* ATCC Nos. 21,114; 21,713; 21,714 or 21,715 or mutants thereof immobilized on a porous phenolic resin wherein said resin has a total surface area of 40 to 200 square meters per gram in the range of pores above 30Å of which at least 30% of the pores have a radius of 30 to 250Å and a water swelling capacity of at least 10% by volume and wherein said system has a binding efficiency of at least 90% when an enzyme load of 500 U/ml carrier is offered or at least 80% when an enzyme load of 1000 U/ml carrier is offered, and an enzyme half-life during isomerization of at least 10 days.

2. The system of claim 1 wherein said porous phenolic resin is an anion exchange type resin.

3. The system of claims 1 or 2 wherein at least 50% of the pores having a radius of 30 to 250Å have a radius of 50 to 150Å.

4. The system of one of claims 1—3 wherein at least 20% of the pores having a radius of 30 to 250Å have a radius of 60 to 90Å.

5. The system of one of claims 1—4 wherein at least 20% of the pores having a radius of 30 to 250Å have a radius of 70 to 80Å.

9

6. A process for the isomerization of glucose comprising contacting a glucose-containing solution with the immobilized glucose isomerase system of claim 1.

7. A process for the isomerization of glucose comprising contacting a glucose-containing solution with the immobilized glucose isomerase system of claim 2.

8. A process for the isomerization of glucose comprising contacting a glucose-containing solution with the immobilized glucose isomerase system of claim 3.

9. A process for the isomerization of glucose comprising contacting a glucose-containing solution with the immobilized glucose isomerase system of claim 4.

10. A process for the isomerization of glucose comprising contacting a glucose-containing solution with the immobilized glucose isomerase system of claim 5.

## Patentansprüche

1. Ein immobilisiertes Gluocoseisomerase-System aus Glucoseisomerase aus Streptomyces olivochromogenes ATCC Nr. 21,114, 21,713, 21,714 oder 21,715 oder Mutanten davon immobilisiert auf einem porösen Phenolharz, welches eine Gesamtoberfläche von 40 bis 200 $m^2/g$ im Porenbereich oberhalb 30Å besitzt, wovon mindestens 30% der Poren einen Radius von 30—250Å aufweisen und das eine Wasserquellkapazität von mindestens 10 Vol.-% besitzt und wobei dieses System eine Bindungseffizienz von mindestens 90% aufweist, wenn eine Enzymbeladung von 500 Einheiten pro ml Träger angeboten wird oder von mindestens 80%, wenn eine Enzymbeladung von 1000 Einheiten pro ml Träger angeboten wird und das eine Enzymhalbwertszeit währand der Isomerisierung von mindestens 10 Tagen aufweist.

2. System gemäß Anspruch 1, worin das poröse Phenolharz ein Anionenaustauscherharz ist.

3. System der Ansprüche 1 oder 2, worin mindestens 50% der Poren mit einem Radius von 30—250Å einen Radius von 50—150Å besitzen.

4. System gemäß einem der Ansprüche 1—3, worin mindestens 20% der Poren mit einem Radius von 30—250Å einen Radius von 60—90Å besitzen.

5. System gemäß einem der Ansprüche 1—4, worin mindestens 20% der Poren mit einem Radius von 30—250Å einen Radius von 70—80Å besitzen.

6. Verfahren zur Isomerisierung von Glucose, wobei eine Glucose enthaltende Lösung mit dem immobilisierten Glucoseisomerase-System gemäß Anspruch 1 in Berührung gebracht wird.

7. Verfahren zur Isomerisierung von Glucose, wobei eine Glucose enthaltende Lösung mit dem immobilisierten Glucoseisomerase-System gemäß Anspruch 2 in Berührung gebracht wird.

8. Verfahren zur Isomerisierung von Glucose, wobei eine Glucose enthaltende Lösung mit dem immobilisierten Glucoseisomerase-System gemäß Anspruch 3 in Berührung gebracht wird.

9. Verfahren zur Isomerisierung von Glucose, wobei eine Glucose enthaltende Lösung mit dem immobilisierten Glucoseisomerase-System gemäß Anspruch 4 in Berührung gebracht wird.

10. Verfahren zur Isomerisierung von Glucose, wobei eine Glucose enthaltende Lösung mit dem immobilisierten Glucoseisomerase-System gemäß Anspruch 5 in Berührung gebracht wird.

## Revendications

1. Système glucose-isomérase immobilisé, comprenant une glucose-isomérase tirée de *Streptomyces clivochromogenes* n° ATCC 21 114, 21 713, 21 714 ou 21 715 ou de mutants de ceux-ci immobilisée sur une résine phénolique poreuse, caractérisé en ce que la résine a une surface totale de 40 à 200 $m^2/g$ dans la gamme de pores de plus de 30Å, parmit lesquels 30% au moins des pores ont un rayon de 30 à 250Å, et une capacité de gonflement par l'eau d'au moins 10% en volume, et en ce que le système a une capacité de fixation d'au moins 90% lorsqu'une charge d'enzyme de 500 U/ml de véhicule lui est offerte ou d'au moins 80% lorsqu'une charge d'enzyme de 1000 U/ml de véhicule lui est offerte, avec une période de demi-vie de l'enzyme au cours de l'isomérisation d'au moins 10 jours.

2. Système selon la revendication 1, caractérisé en ce que la résine phénolique poreuse est une résine du type échangeuse d'anions.

3. Système selon la revendication 1 ou 2, caractérisé en ce qu'au moins 50% des pores ayant un rayon de 30 à 250Å ont un rayon de 50 à 150Å.

4. Système selon l'une quelconque des revendications 1 à 3, caractérisé en ce qu'au moins 20% des pores ayant un rayon de 30 à 250Å ont un rayon de 60 à 90Å.

5. Système selon l'une quelconque des revendications 1 à 4, caractérisé en ce qu'au moins 20% des pores ayant un rayon de 30 à 250Å ont un rayon de 70 à 80Å.

6. Procédé pour l'isomérisation de glucose, caractérisé en ce qu'il consiste à mettre une solution contenant du glucose en contact avec la système glucose-isomérase immobilisé de la revendication 1.

7. Procédé pour l'isomérisation de glucose, caractérisé en ce qu'il consiste à mettre une solution contenant du glucose en contact avec le système glucose-isomérase immobilisé de la revendication 2.

8. Procédé pour l'isomérisation de glucose, caractérisé en ce qu'il consiste à mettre une solution contenant du glucose en contact avec le système glucose-isomérase immobilisé de la revendication 3.

9. Procédé pour l'isomérisation de glucose, caractérisé en ce qu'il consiste à mettre une solution contenant du glucose en contact avec le système glucose-isomérase immobilisé de la revendication 4.

10. Procédé pour l'isomérisation de glucose, caractérisé en ce qu'il consiste à mettre une solution contenant du glucose en contact avec le système glucose isomérase immobilisé de la revendication 5.